**Europäisches Patentamt**

**European Patent Office**

**Office européen des brevets**

(19)

(11) Veröffentlichungsnummer : **0 081 051 B2**

(12)

# NEUE EUROPÄISCHE PATENTSCHRIFT

(45) Veröffentlichungstag der neuen Patentschrift :
**17.04.91 Patentblatt 91/16**

(51) Int. Cl.$^5$ : **A61B 17/22**

(21) Anmeldenummer : **82108967.9**

(22) Anmeldetag : **28.09.82**

(54) Auslösevorrichtung für Stosswellen zu therapeutischen Zwecken.

(30) Priorität : **25.11.81 DE 3146628**

(43) Veröffentlichungstag der Anmeldung :
**15.06.83 Patentblatt 83/24**

(45) Bekanntmachung des Hinweises auf die
Patenterteilung :
**12.02.86 Patentblatt 86/07**

(45) Bekanntmachung des Hinweises auf die
Entscheidung über den Einspruch :
**17.04.91 Patentblatt 91/16**

(84) Benannte Vertragsstaaten :
**CH FR GB IT LI NL SE**

(56) Entgegenhaltungen :
**CH-A- 446 610**
**CH-A- 528 902**
**DE-A- 2 610 061**
**DE-A- 3 119 295**
**DE-B- 2 351 247**
**DE-B- 2 418 631**
**DE-C- 856 788**
**DE-C- 2 538 960**
**DE-C- 2 635 635**
**FR-A- 2 247 195**
**GB-A- 1 436 032**
**US-A- 3 129 704**
**US-A- 3 566 645**
**US-A- 3 835 845**
**US-A- 3 942 531**
**US-A- 4 031 884**
**US-A- 4 191 189**
**Electronique Applications, No.5, 1978, D. Heyden, "La défibrillation électrique,pp.5-8**
**Academic Press,1976, D.W.Hill and A.M. Dolan, "Intensive care instrumentation",**
**pp.213-216**
**Acta phys. Scandinav., Vol. 33, 1954, A.Carlsten et al., "The Electrocardiogramm of Rabbits in Blast Injury"pp. 243-256**
**Acta phys. Scandinav., 1955, pp.119-126, C.-J.Clemedson et al., "Propagation of High Explosive Air Shock Wave Through Diffenrent parts of an Animal Body"**
**Revue Générale de l'Electricité, Bd. 71, no. 11, Nov. 1962, pp.471-476**
**Electrical Engineering Transactions. April 1972, Keefe et al., pp.9-13**

(56) Entgegenhaltungen :
**Revue International EEE Spectrum, Februar 1969, Dalziel et al., pp.44-50**
**Basic Research Cordiology, 77, 1982, pp.237-249**
**Münchner Medizinische Wochenzeitschrift, 125, 1983,No. 8, Chaussy et al, pp.151-155**
**Journal of Urology, Bd. 127, 1982,pp.417-420**
**Radiographic Atlas of the Genitoury System, Ney C. Friedenberg RM (eds.), J. B. Lippincott Co. 1981, p.10. Chapter 1**
**Radiology of the Urinary System. Elkin M (ed.); Littel, Brown & Co., Boston 1980, p. 8 Chapter 1**
**Roentgen Diagnosis of the Urogenital System, Olsson O.(ed.),Springer-Verlag, New Yorl 1973. pp.10-11, part 1**
**Dtsch. Z.Cir.(1936),247.411, Hilgenfeldt O. "Das Veratmungspyelogram"**
**J.Urol.(1939), 42:4, 381, Hess E. "Respiration pyelography as an aidin diagnosis"**
**Amer. J. Roentgenol.(1940), 44:71, Bacon RD. "Respiratino pyelography"**
**Medizin in unserer Zeit, 4.Jahrgang, 1980, No.1, pp.10-14**

(73) Patentinhaber : **DORNIER GMBH**
**Postfach 1420**
**W-7990 Friedrichshafen 1 (DE)**

(72) Erfinder : **Forsmann, Bernd, Dr.**
**Salemweg 6**
**W-7990 Friedrichshafen 1 (DE)**
Erfinder : **Hepp, Wolfgang, Dr.**
**Hardtstrasse 6**
**W-7997 Immenstaad (DE)**
Erfinder : **van Ackern, Klaus, Prof. Dr.**
**Adalbertweg 10**
**W-8035 Gauting (DE)**
Erfinder : **Chaussy, Christian, Prof. Dr.**
**Friedenstrasse 13a**
**W-8034 Germering (DE)**

(74) Vertreter : **Landsmann, Ralf, Dipl.-Ing.**
**c/o DORNIER GMBH Postfach 1420**
**W-7990 Friedrichshafen 1 (DE)**

EP 0 081 051 B2

### Beschreibung

Die Erfindung betrifft eine Auslösevorrichtung zur berührungungsfreien Zerkleinerung von Konkrementen im menschlichen Körper.

Bekannt ist ein Gerät zur berührungslosen Zerkleinerung von Konkrementen in Körpern von Lebewesen mittels Stosswellen (DE-PS-2 351 247). Dabei werden ausserhalb des Körpers durch elektrische Entladungen einer Unterwasserfunkenstrecke in einer Fokussierungskammer Stosswellen erzeugt, die auf das Konkrement fokussiert werden und dieses zerkleinen. Diese Stosswellen durchdringen das Körpergewebe ohne Schädigungen zu verursachen. Möglich ist dabei allerdings die Auslösung von Extrasystolen in der erregbaren Phase des Herzschlages. Bei kreislaufgefährdeten Patienten kann dadurch der Herzrhythmus gestört werden. Eine ausreichende Blutumwälzung ist dann nicht mehr gewährleistet. Zusätzlich ist bei der Wahl des Auslösezeitpunktes darauf zu achten, dass die Koordinaten des Zielobjektes, z. B. der Niere, die durch die Atmung periodisch geändert werden, im Augenblick der Stossauslösung möglichst konstant sind, um das Konkrement genau zu treffen.

Aus der CH-A-528 902 ist ein Apparat zur Stützung des Kreislaufs bekannt, der den Körper des Patentien periodisch hin- und herbewegt. Diese Pumpbewegung wird mit dem natürlichen Herszchlag synchronisiert, damit sich beide Wirkungen verstärken und nicht gegenseitig aufheben.

Aus der DE-B2-2 418 631 ist ein Herzstimulator bekannt, bei dem ein stillstehendes oder zu langsam schlagendes Herz durch Druckwellen mechanisch angeret wird. Die Druckwellen werden periodisch (mit der Frequenz des natürlichen Pulses) erzeugt, damit sich nicht die Wirkungen der äusseren Stimulation und die der natürlichen Anregung aufheben.

Die beiden letztgenanten Literaturstellen liegen auf dem Gebiet der Herz- und Kreislaufbehandlungen, sie lehren die bei der Behandlung des Herzens selbstverständliche Berücksichtigung des natürlichen Pulses.

Auf dem Gebiet der Erfindung – der Steinzertrümmerung, dem Einwirken auf leblose Materie – ist eine Berücksichtigung periodischer Körperfunktionen nicht bekannt. Weder in der Urologie (Nierensteinbehandlung) noch auf dem Gebiet der Verdauungskrankheiten (Gallensteinbehandlung) wurden bisher die davon unabhängigen Herz- und kreislauffunktionen berücksichtigt.

Der Erfindung liegt die Aufgabe zugrunde, eine Vorrichtung zur Steinzertrümmerung so auszubilden, dass Körperfunktionen nicht gestört werden oder die Effektivität der Stosswellenbehandlung verbessert wird.

Die Aufgabe wird erfindungsgemäss gelöst durch eine Vorrichtung mit den im Anspruch 1 oder 3 genannten Merkmalen, Weitere Ausgestaltungen der Erfindung sind in den Unteransprüchen angegeben.

Ein Vorteil dieser Erfindung ist, dass auch bei kreislaulgefährdeten Patienten Stosswellen angewandt werden können und dadurch auch bei dieser Patientengruppe auf operative Eingriffe verzichtet werden kann. Die automatische zeitliche Korrelation mit dem Herzschlag oder mit weiteren Körperfunktionen nimmt dem behandelnden Arzt die Aufgabe ab, die Applikation der Stosswellen den Körperrhythmen anzupassen. Fehlentscheidungen können dadurch vermieden werden.

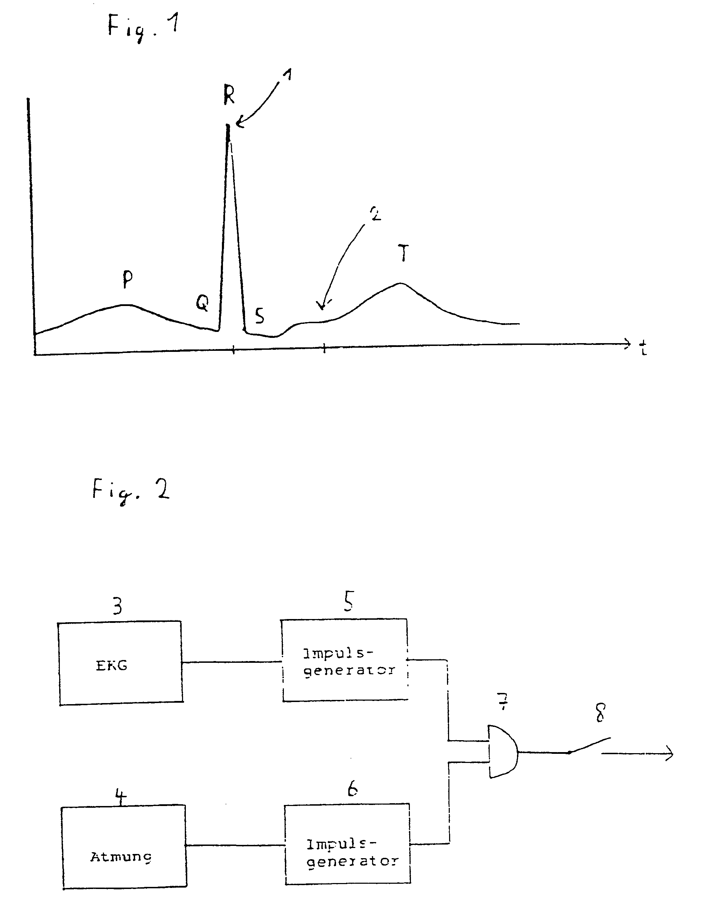

Anhand zweier Figuren wird die Erfindung näher beschrieben. Die Figur 1 zeigt den zeitlichen Verlauf der Spannungen des EKG bei normalen Herzschlag. Die Bezeichnungen P, Q, R, S, T für die Zacken sind in der Medizin gebräuchlich. Der steilste Anstieg ist die R-Zacke (Punkt 1). Punkt 2 zeigt einen gefahrlosen Auslösezeitpunkt für eine Stosswellenapplikation. Bei einer einkanaligen Ausführung wird das EKG in bekannter Weise aufgenommen, aus dem Anstieg der R-Zacke 1 wird in ebenfalls bekannter Weise ein Triggerimpuls gewonnen und auf eine Verzögerungseinrichtung geschickt. Diese ist im Bereich von 0 bis 1000 ms einstellbar und gibt nach der vom Arzt vorgewählten Zeit (Abstand 1-2) einen Auslöseimpuls über einen Freigabeschalter auf die Funkenstrecke, wodurch diese zündet und eine Stosswelle erzeugt.

Die Fig. 2 zeigt das Blockschaltbild einer Vorrichtung πt weiterehender Automatisierung durch Berücksichtigung einer zweiten Körperfunktion, hier der Atmung, in einem zweiten Kanal. Zwei Meßwertaufnehmer, Aufnehmer 3 für das EKG, Aufnehmer 4 für die Atembewegung, geben ihre Meßwerte an je einen Impulsgenerator 5, 6. Diese Generatoren 5, 6 enthalten an sich bekannte Einrichtungen zum Gewinnen eines Triggerimpulses und zur einstellbaren Verzögerung, so dass sie auf eine bestimmte Phase der Meßwerte hin, z. B. 10 ms nach dem Auftreten der R-Zacke oder im Moment des Atemstillstandes, einen Impuls geben. Die Impulsgeneratoren sind an die Eingänge eines «und»-Gatters 7 angeschlossen. Der Ausgang des Gatters 7 führt über einen manuellen Freigabe Schalter 8 zur Funkenstrecke.

Ein Auslöseimpuls erreicht die Funkenstrecke in diesem Beispiel nur, wenn

a) der Arzt den Schalter 8 geschlossen hält,

b) die Atmungsbewegung gerade ruht,

c) die R-Zacke des EKG gerade vor 10 ms aufgetreten ist.

Die Berücksichtigung weiterer Körperfunktionen durch weitere parallele Kanäle, das schnell aufeinanderfolgende Auslösen mehrerer Stosswellen durch schnell aufeinanderfolgende.

## Ansprüche

1. Auslösevorrichtung für Stosswellen zur berührungsfreien Zerkleinerung von Konkrementen, gekennzeichnet durch
   a) einen Messwertaufnehmer für den Herzschlag
   b) eine Schaltung, die aus einem Extremwert des vom Messwertaufnehmer gelieferten Meßsignals einen Triggerimpuls gewinnt, und
   c) eine einstellbare Verzögerungseinrichtung, die den Triggerimpuls zeitlich verzögert über einen manuell betätigten Freigabeschalter an die Stosswellenquelle weiterleitet.

2. Auslösevorrichtung nach Anspruch 1, dadurch gekennzeichnet, dass als Messwertaufnehmer eine EKG-Sonde verwendet wird, wobei der Extremwert die R-Zacke des EKG ist.

3. Auslösevorrichtung für Stosswellen zur berührungsfreien Zerkleinerung von Konkrementen, gekennzeichnet durch
   a) einen Messwertaufnehmer für die Atmung,
   b) eine Schaltung, die aus einem Extremwert des vom Messwertaufnehmer gelieferten Meßsignals einen Triggerimpuls gewinnt, und
   c) eine einstellbare Verzögerungseinrichtung, die den Triggerimpuls zeitlich verzögert über einen manuell betätigten Freigabeschalter an die Stosswellenquelle weiterleitet.

4. Auslösevorrichtung nach Anspruch 3, dadurch gekennzeichnet, dass als Messwertaufnehmer ein Spirometer verwendet wird, wobei als Extremwert die Phase des ausgeatmeten Zustands verwendet wird.

5. Auslösevorrichtung nach einem der Ansprüche 1 bis 4 dadurch gekennzeichnet, dass die Schaltung aus jedem zweiten, dritten usw. Extremwert einen Triggerimpuls gewinnt.

6. Auslösevorrichtung für Stosswellen nach den Ansprüchen 1 und 3 gekennzeichnet durch Messkanäle für den Herzschlag und die Atmung, die jeweils aufweisen
   a) einen Messwertaufnehmer für eine der Körperfunktionen,
   b) eine Schaltung, die aus einem Extremwert des vom Messwertaufnehmer gelieferten Meßsignals einen Triggerimpuls gewinnt,
   c) eine einstellbare Verzögerungseinrichtung, die den Triggerimpuls zeitlich verzögert abgibt und durch ein «UND»-Gatter, an das die Ausgänge aller Messkanäle angeschlossen sind, welches beim gleichzeitigen Eintreffen der Triggerimpulse aller Kanäle einen Impuls zur Auslösung einer Stosswelle über einen manuell betätigten Freigabeschalter an die Stosswellenquelle abgibt.

## Claims

1. Apparatus for triggering shock waves for the contact-free comminution of concretions, characterised by :
   a) a measured value pick-up for the heart beat,
   b) a circuit which obtains a trigger pulse from an extreme value of the measuring signal supplied by the measured value pick-up, and
   c) an adjustable delay device which passes the trigger pulse with a time delay to the shock wave source via a manually actuated release switch.

2. Trigger apparatus according to claim 1, characterised in that the measured value pick-up used is an ECG probe, the extreme value being the R-blip of the ECG.

3. Apparatus for triggering shock waves for the contact-free comminution of concretions, characterised by :
   a) a measured value pick-up for respiration,
   b) a circuit which obtains a trigger pulse from an extreme value of the measuring signal supplied by the measured value pick-up, and
   c) an adjustable delay device which passes the trigger pulse with a time delay to the shock wave source via a manually actuated release switch.

4. Trigger apparatus according to claim 3, characterised in that a spirometer is used as the measured value pick-up, the exhaled condition phase being used as the extreme value.

5. Trigger apparatus according to one of claims 1 to 4, characterised in that the circuit obtains a trigger pulse from every second, third, etc,, extreme value.

6. Apparatus for triggering shock waves according to claims 1 and 3, characterised by channels for measuring the heart beat and respiration, and each having :
   a) a measured value pick-up for one of the body functions,
   b) a circuit which obtains a trigger pulse from an extreme value of a measuring signal aupplied by the measured value pick-up,
   c) an adjustable delay device which emits the trigger pulse with a timing delay,
   and by an "AND" gate to which the outputs of all measuring channels are connected and which, upon simultaneous arrival of trigger pulses from all channels, delivers to the shock wave source a shock wave via a manually actuated release switch.

## Revendications

1. Dispositif de déclenchement d'ondes de choc pour pulvériser, sans contact, des concrétions, caractérisé par le fait qu'il comporte
   a) un capteur de valeurs de mesure pour les battements cardiaques,
   b) un circuit permettant d'obtenir une impulsion de déclenchement à partir d'une valeur extrême du signal de mesure délivré par le capteur de valeurs de mesure, et

c) un dispositif de retardement réglable, qui retransmet à la source d'ondes de choc l'impulsion de déclenchement retardée dans le temps, par l'intermédiaire d'un commutateur de déblocage actionné manuellement.

2. Dispositif de déclenchement selon la revendication 1, caractérisé en ce que l'on utilise comme capteur de valeurs de mesure une sonde pour électrocardiogramme, la valeur extrême étant fournie par la pointe R de l'électrocardiogramme.

3. Dispositif de déclenchement d'ondes de choc pour pulvériser, sans contact, des concrétions, caractérisé par le fait qu'il comporte

a) un capteur de valeurs de mesure pour la respiration,

b) un circuit permettant d'obtenir une impulsion de déclenchement à partir d'une valeur extrême du signal de mesure délivré par le capteur de valeurs de mesure, et

c) un dispositif de retardement réglable, qui retransmet à la source d'ondes de choc l'impulsion de déclenchement retardée dans le temps, par l'intermédiaire d'un commutateur de déblocage actionné manuellement.

4. Dispositif de déclenchement selon la revendication 3, caractérisé en ce que l'on utilise comme capteur de valeurs de mesure un spiromètre, et qu'on utilise comme valeur extrême la phase de l'état d'expiration.

5. Dispositif de déclenchement selon l'une des revendications 1 à 4, caractérisé en ce que le circuit établit une impulsion de déclenchement à chaque seconde, troisième, etc. valeur extrême.

6. Dispositif de déclenchement d'ondes de choc, selon les revendications 1 et 3 caractérisé par des canaux de mesure pour les battements cardiaques et la respiration, qui comportent chacun

a) un capteur de valeurs de mesure pour l'une des fonctions du corps,

b) un circuit qui établit une impulsion de déclenchement à partir d'une valeur extrême du signal de mesure délivré par le capteur de valeurs de mesure,

c) un dispositif de retardement réglable, qui délivre de façon retardée dans le temps l'impulsion de déclenchement, et

par une porte "ET", à laquelle sont raccordées les sorties de tous les canaux de mesure et qui, lors de l'apparition simultanée des impulsions de déclenchement de tous les canaux, envoie à la source d'ondes de choc, par l'intermédiaire d'un commutateur de déblocage actionné manuellement, une impulsion pour déclencher une onde de choc.

## Fig. 1

## Fig. 2